Europäisches Patentamt

European Patent Office

Office européen des brevets

(19)

(11) Veröffentlichungsnummer: **0 045 388**
**B1**

(12) # EUROPÄISCHE PATENTSCHRIFT

(45) Veröffentlichungstag der Patentschrift:
31.10.84

(21) Anmeldenummer: 81105303.2

(22) Anmeldetag: 08.07.81

(51) Int. Cl.³: **A 61 B 5/08**

(54) **Gerät zur Untersuchung der Lungenfunktion.**

(30) Priorität: 31.07.80 DE 3029155

(43) Veröffentlichungstag der Anmeldung:
10.02.82 Patentblatt 82/6

(45) Bekanntmachung des Hinweises auf die Patenterteilung:
31.10.84 Patentblatt 84/44

(84) Benannte Vertragsstaaten:
BE CH DE FR GB IT LI NL

(56) Entgegenhaltungen:
DE - A - 1 466 835
DE - A - 1 918 566
FR - A - 1 508 303

MEDICAL AND BIOLOGICAL ENGINEERING AND
COMPUTING, Band 17, Nr. 4, Juli 1979, Seiten 536-538,
S. JACOBSEN et al.: "Technical note - Switching device
for alternation between expiratory and inspiratory
flowmeters, incorporating a delay for synchronisation of
respiratory flowmeter and mass spectrometer signals"

(73) Patentinhaber: Erich Jaeger GmbH & Co. K.G.,
D-8706 Höchberg (DE)

(72) Erfinder: Dorsch, Albin, Margetshöchheimer Strasse 125,
D-8702 Zell (DE)

(74) Vertreter: Von Bezold, Dieter, Dr. Patentanwälte
Dr.Dieter v. Bezold et al, Dipl.-Ing. Peter Schütz Dipl.-Ing.
Wolfgang Heusler
Postfach 860260 Maria-Theresia-Strasse 22,
D-8000 München (DE)

## Beschreibung

Die vorliegende Erfindung betrifft ein Gerät zur Untersuchung der Lungenfunktion mit einem an die Atemwege einer zu untersuchenden Person anschließbaren Atemrohr, das mit einem Strömungssensor gekoppelt und über ein Ventil, das eine Offenstellung und eine Schließstellung aufweist, an eine Atemluftleitung angeschlossen ist.

Ein Gerät dieser Art ist aus DE-A-1 918 566 bekannt. Dieses bekannte Gerät enthält einerseits passive Rückschlagventile, die als Inspirations- und Expirations-Ventil arbeiten sowie aktiv gesteuerte Magnetventile. Eines dieser Magnetventile wird durch einen Strömungssensor bei Beginn der Ausatmung geöffnet, um einen dem Totraum entsprechenden Teil der Expirationsluft durch einen Auslaß abströmen zu lassen. Das Magnetventil wird wieder geschlossen, nachdem eine bestimmte Luftmenge ausgeatmet worden ist, was mit Hilfe der Strömungsmeßvorrichtung ermittelt wird.

Es ist ferner aus DE-A-1 466 835 bekannt, vom Druckabfall, der an einem in den Atemluftweg geschalteten Strömungswiderstand entsteht, ein elektrisches Signal abzuleiten, das anzeigt, ob der Proband einatmet (Inspirationsphase) oder ausatmet (Expirationsphase).

Aus der Zeitschrift J. Appl. Physiol. 19(3), 534—535, 1964 ist es bekannt, das mit den Atemwegen eines Probanden verbundene Atemrohr nicht direkt mit einem Strömungssensor (Pneumotachograph) zu koppeln, sondern über dünnwandige Beutel, die sich in einem luftdicht verschlossenen Kasten befinden (»Bag-in-Box-System«).

Alle bekannten Geräte zur Untersuchung der Lungenfunktion, bei denen das mit den Atemwegen des Probanden verbundene Atemrohr über ein Ventilsystem mit getrennten Inspirationsluft- und Expirationsluft-Leitungen verbunden sind, lassen in der einen oder anderen Weise zu wünschen übrig. Bei Geräten der aus der DE 1 918 566 B2 bekannten Art, deren Ventilsystem Rückschlagventile enthält, die durch den Druck der Atemluft direkt betätigt werden, tritt ein unerwünscht hoher Druckabfall an diesen Ventilen auf. Bei Geräten, deren Ventilsystem durch ein vom Druck der Atemluft abgeleitetes Signal indirekt und mit Fremdenergie gesteuert wird (DE 1 915 959 B2) ist wegen der schwankenden und relativ kleinen Werte des Druckes an den Grenzen der jeweiligen Atemphasen eine sichere und eindeutige Ventilbetätigung nicht mit Sicherheit zu erreichen.

Der vorliegenden Erfindung liegt dementsprechend die Aufgabe zugrunde, ein Gerät der eingangs genannten Art derart weiterzubilden, daß eine sichere und eindeutige Betätigung des Ventilsystems bei gleichzeitig geringstmöglichem Strömungswiderstand im Offenzustand gewährleistet ist.

Die Erfindung löst diese Aufgabe bei einem Gerät der eingangs genannten Art dadurch, daß die Offenstellung des Ventils durch einen Drucksensor, der auf den Druck im Atemrohr anspricht, beim Überschreiten eines vorgegebenen Mindestdruckwertes eingestellt wird und die Schließstellung durch den Strömungssensor beim Unterschreiten eines vorgegebenen Strömungsweges eingestellt wird.

Weiterbildungen und vorteilhafte Ausgestaltungen des erfindungsgemäßen Geräts sind Gegenstand von Unteransprüchen.

Bei dem erfindungsgemäßen Gerät ist ein eindeutiges Öffnen und Schließen jedes Ventils und damit eine eindeutige Definition der jeweiligen Atemphase (Inspiration, Expiration) gewährleistet. Der Strömungsweg vom Atemrohr über ein geöffnetes Ventil zu einer Atemluftleitung hat einen geringen Strömungswiderstand auch im Falle von Ventilsystemen, die ein Atemrohr mit mehr als zwei Atemluftleitungen zu verbinden gestattet. Die Ventilbetätigung ist außerdem schnell und sicher. Um eine Ventilbetätigung zu Beginn der Inspirations- und Expirationsphase auch schon bei sehr kleinen Druckunterschieden bezüglich des Ruhe- oder Umgebungsdruckes zu gewährleisten, wird außerdem ein besonders empfindlicher Druckaufnehmer angegeben.

Im folgenden wird ein Ausführungsbeispiel der Erfindung unter Bezugnahme auf die Zeichnung näher erläutert. Es zeigt

Fig. 1 eine schematische, vereinfachte Darstellung einer Ausführungsform des vorliegenden Gerätes;

Fig. 2 eine mehr ins einzelne gehende Darstellung des Gerätes gemäß Fig. 1;

Fig. 3 ein Schnitt in einer Ebene III-III der Fig. 2; und

Fig. 4 eine schematische Schnittdarstellung eines vorteilhaften Druckaufnehmers, der sich besonders für Geräte der vorliegenden Art eignet.

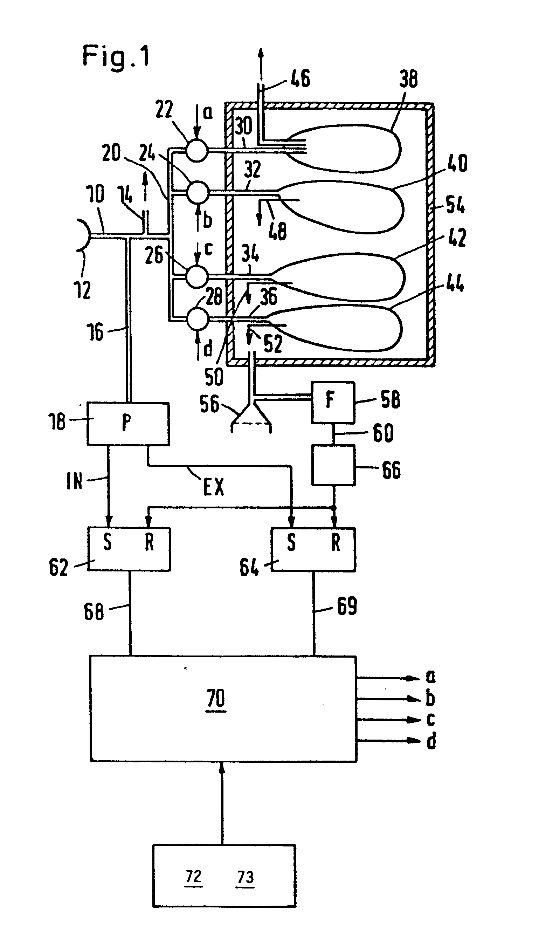

Das in Fig. 1 schematisch dargestellte Gerät enthält ein Atemrohr 10, das über eine Anschlußvorrichtung 12, z. B. ein Mundstück oder eine Atemmaske, an die Atemwege einer zu untersuchenden Person (»Proband«) anschließbar ist. Das Atemrohr 10 ist mit einer Anschlußleitung 14 zur Entnahme von Atemluftproben und mit einer weiteren Anschlußleitung 16 verbunden, die zu einem Drucksensor 18 führt. An das Atemrohr 10 ist ferner ein Verteiler 20 angeschlossen, der über Ventile 22, 24, 26 und 28 mit entsprechenden Atemluftleitungen 30, 32, 34 bzw. 36 verbunden ist. An das den Ventilen abgewandte Ende jeder Atemluftleitung ist ein dünnwandiger Beutel 38, 40, 42 bzw. 44, z. B. aus Gummi oder Kunststoffolie, angeschlossen. In den Beuteln 38, 40, 42 und 44 mündet ferner jeweils eine Entnahmeleitung 46, 48, 59 bzw. 52, durch die das Gas aus dem jeweiligen Beutel zu Analysezwecken oder dgl. entnommen werden kann.

Die Beutel 38, 40, 42 und 44 sind in einem luftdicht verschließbaren Kasten 54 angeordnet, dessen Inneres über ein Pneumotachographenrohr 56 mit der Umgebung in Verbindung steht. Das Pneumotachographenrohr 56 enthält, wie üblich, einen Strömungswiderstand und ist mit einem Differenzdrucksensor 58 gekoppelt, der auf einer Ausgangsleitung 60 ein der Atemluftströmung (Luftmenge/Zeiteinheit) entsprechendes Ausgangssignal liefert.

Der Drucksensor 18 liefert ein Ausgangssignal, das hinsichtlich Betrag und Vorzeichen dem im Atemrohr 10 herrschenden Druck entspricht. Um die prinzipielle Arbeitsweise des Gerätes gemäß Fig. 1 klarer darzustellen, ist der Drucksensor 18 mit zwei Ausgangsleitungen dargestellt und es soll angenommen werden, daß auf der mit »IN« bezeichneten Leitung ein Ausgangssignal auftritt, wenn der Proband einatmet, während auf einer mit »EX« bezeichneten Ausgangsleitung ein Signal auftritt, wenn der Proband ausatmet.

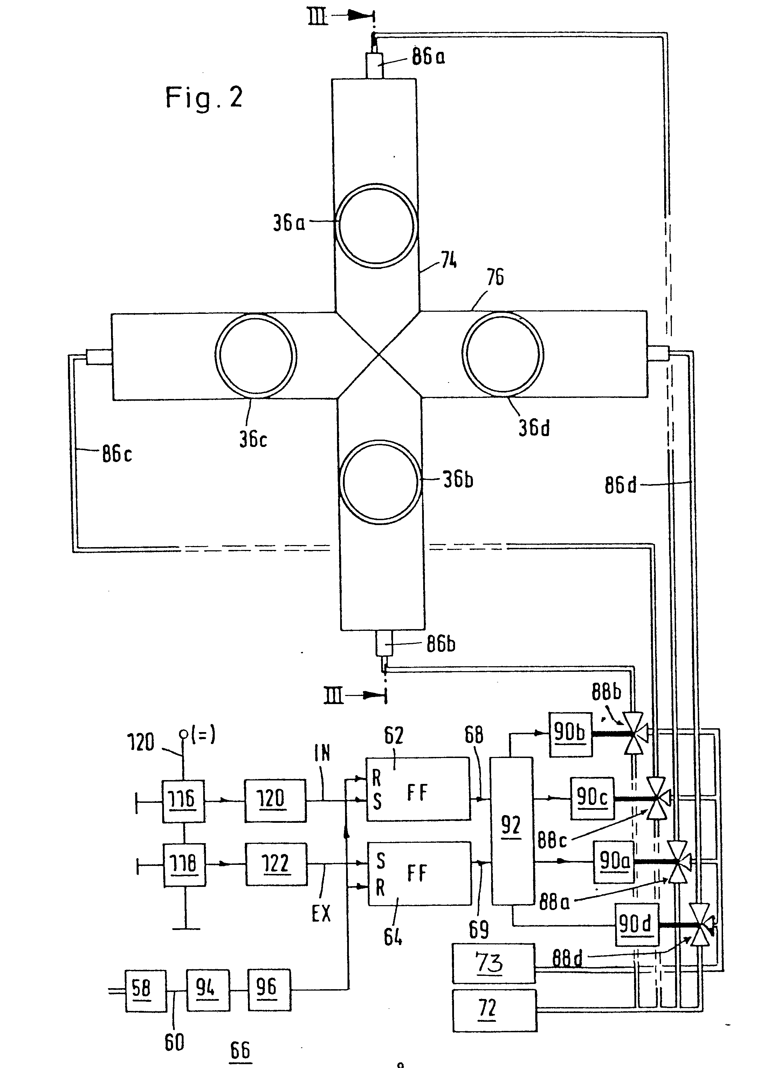

Die IN-Leitung ist mit dem Setzeingang S eines Flipflops 62 gekoppelt. Die EX-Leitung ist mit dem Setzeingang S eines Flipflops 64 gekoppelt. Das Ausgangssignal des Differenzdrucksensors 58 wird über die Leitung 60 einer Schaltungsanordnung 66 zugeführt, die einen Ausgangsimpuls liefert, wenn die Atemluftströmung Null wird oder unter einen bestimmten, kleinen Schwellenwert fällt. Das Ausgangssignal der Schaltungsanordnung 66 wird den Rücksetzeingängen R der Flipflops 62 und 64 zugeführt. Die Flipflops 62 und 64 liefern jeweils im gesetzten Zustand ein Ausgangssignal. Die Ausgangssignale werden über Leitungen 68 und 69 einer vereinfacht als Block dargestellten Steuereinrichtung 70 zugeführt. Die Steuereinrichtung 70 steuert die Zufuhr von Druckluft bzw. Vakuum von einer Druckluftquelle 72 bzw. Vakuumquelle 73 zu Steuereingängen a, b, c und d der Ventile 22, 24, 26 bzw. 28 entsprechend einem gewünschten Programm oder einer gewünschten Einstellung. Die Steuereinrichtung 70 ermöglicht es, jedes der Ventile 22, 24, 26 und 28 während einer gewünschten Atemphase zu öffnen oder zu schließen. Weitere Einzelheiten werden im folgenden anhand der Fig. 2 bis 4 erläutert.

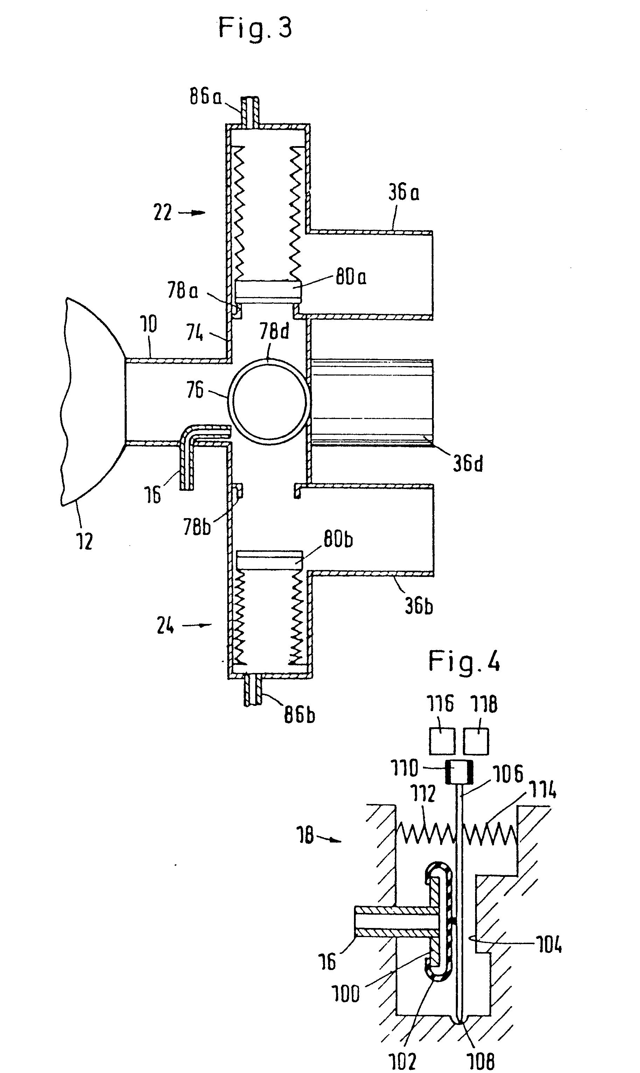

In den Fig. 2 und 3 ist eine vorteilhafte Ausgestaltung des Ventil- und Leitungssystems für die Atemluft dargestellt. Für gleiche Teile sind die gleichen Bezugszeichen wie in Fig. 1 verwendet. Das Atemrohr 10 ist mit der Kreuzungsstelle zweier quer verlaufenden Verteilerrohre 74 und 76 verbunden, von denen wiederum jeweils zwei Atemluftleitungen 36a, 36b bzw. 36c und 36d in der gleichen Richtung wie das Atemrohr 10, jedoch bezüglich dieses versetzt abzweigen. Beidseits des Kreuzungspunktes und nahe bei diesem enthält jedes Verteilerrohr 74, 76 zwei Ventilsitze, von denen in Fig. 3 die Ventilsitze 78a, 78b und 78d zu sehen sind. Die Ventilsitze arbeiten mit Schließkörpern 80a bzw. 80b usw. zusammen, die jeweils durch einen flexiblen Balgen 84a bzw. 84b geführt und mit dem jeweils benachbarten, geschlossenen Ende des betreffenden Verteilerrohrs verbunden sind. Im Inneren der Balgen mündet jeweils eine pneumatische Steuerleitung 86a, 86b, 86c und 86d. Die Steuerleitungen sind über Dreiwege-Magnetventile 88a, 88b, 88c bzw. 88d mit der Druckluftquelle 72 und der Vakuumquelle 73 (Fig. 2) verbunden (Fig. 2). Beim Öffnen des Ventils wird die betreffende Steuerleitung mit der Vakuumquelle 73, beim Schließen mit der Druckluftquelle 72 verbunden, so daß eine sehr schnelle Ventilbetätigung gewährleistet ist. Die Magnetspulen 90a bis 90d der Magnetventile sind mit einer Schaltvorrichtung verbunden, die z. B. Mehrfach-Umschalter oder eine Art von Kreuzschienenverteiler enthalten kann und es gestattet, eine gewünschte der Ausgangsleitungen 68, 70 mit einem gewünschten Magnetventil zu verbinden.

In Fig. 3 ist das den Ventilsitz 78a und den Schließkörper 80a enthaltende Ventil, das z. B. dem Ventil 22 in Fig. 1 entspricht, in der Schließstellung und das den Ventilsitz 78b sowie den Schließkörper 80b enthaltende Ventil, das z. B. dem Ventil 24 in Fig. 1 entspricht, in der Offenstellung dargestellt.

Die das Rücksetzsignal liefernde Schaltungsanordnung 66 kann z. B. eine Schwellenwertschaltung 94 und einen nachgeschalteten Inverter 96 enthalten.

Fig. 4 zeigt eine besonders vorteilhafte Ausgestaltung des Druckaufnehmers 18. Die Anschlußleitung 16 mündet in der Mitte einer Halterungs- und Anschlagplatte 100, mit deren Rand eine dünnwandige Gummimembrane 102 verbunden ist. Der Anschlagplatte 100 liegt ein Anschlag 104 gegenüber. Mit der Gummimembrane 102 ist, z. B. durch einen Tropfen Kleber, ein Schalthebel 106 verbunden, dessen eines Ende durch eine Schneidenlagerung 108 gelagert ist und dessen anderes Ende einen kleinen Permanentmagneten 110 trägt. Durch zwei schwache Federn 112, 114 wird der Schalthebel 106 in der dargestellten Ruhelage gehalten, in der er sich etwa in der Mitte zwischen der Anschlagplatte 100 und dem Anschlag 104 befindet.

Dem Permanentmagneten 100 liegen zwei Hall-Platten 116, 118 symmetrisch gegenüber, die in üblicher Weise mit einem Gleichstrom-Erregungskreis 120 verbunden sind (siehe Fig. 2) und jeweils einen Signalausgang aufweisen, der über eine Verstärker- oder Schwellenwertschaltung 120 bzw. 122 mit der IN-Leitung bzw. EX-Leitung gekoppelt ist.

Schon bei sehr geringem Über- oder Unterdruck in der Anschlußleitung 16, z. B. Drücken von weniger als 10 bis 20 Pa (1 bis 2 mm WS) wird der Schalthebel 106 ausgelenkt, so daß das Magnetfeld des Permanentmagneten 110 die eine oder die andere Hall-Platte 116 oder 118 stärker erregt. Die Ausgangsspannung der betreffenden Hall-Platte betätigt dann z. B. einen Schmitt-Trigger in der zugehörigen Schwellenwert- und Verstärkerschaltung 120, 122, so daß ein das Flipflop 62 oder 64 setzendes Ausgangssignal auf der Leitung IN oder EX auftritt, je nachdem ob der Druck in der Anschlußleitung 16 kleiner oder größer als der Umgebungsdruck ist. Das gesetzte Flipflop bewirkt dann über den Verteiler in der Schaltvorrichtung 92 die Betätigung des gewünschten Magnetventils, das den erforderlichen Steuerdruck zum Öffnen des betreffenden Ventils 22, 24, 26 oder 28 bewirkt. Das geöffnete Ventil wird durch das gesetzte Flipflop unabhängig vom Druck in der Anschlußleitung 16 so lange offengehalten, bis die Atemluftströmung auf Null abgefallen ist und die Schaltungsanordnung 66 ein Rücksetzsignal für das Flipflop liefert.

Wenn ein Ventil auf Inspiration und das andere auf Expiration geschaltet ist, laufen also bei einem Atemzug die folgenden Schaltvorgänge ab:

Anfangs sind alle Ventile geschlossen.

Wenn der Proband nun ausatmen will, entsteht im Atemluftverteiler und damit in der Anschlußleitung 16 ein Überdruck, so daß der Drucksensor 18 anspricht, das Flipflop 64 gesetzt und das für die Expirationsluft vorgesehene Ventil geöffnet wird. Der Proband kann nun ausatmen.

Der Proband atmet aus, bis die Strömungsgeschwindigkeit der Atemluft auf Null absinkt. Nun wird ein Rücksetzsignal für das gesetzte Flipflop erzeugt und das Expirationsventil wird geschlossen.

Der Proband will nun einatmen. Im Atemluftverteiler sowie der Anschlußleitung 16 entsteht ein Unterdruck und das auf Inspiration geschaltete Ventil wird in der oben beschriebenen Weise geöffnet.

Der Proband kann einatmen, während das Inspirationsventil durch das gesetzte Flipflop offengehalten wird.

Der Proband atmet, bis die Strömungsgeschwindigkeit der Inspirationsluft auf Null absinkt. Dies bewirkt das Schließen des Inspirationsventils.

Da die Atemluftventile infolge ihrer Ausgestaltung und pneumatischen Betätigung sowie der hohen Empfindlichkeit des Drucksensors 18 praktisch verzögerungsfrei ansprechen, tritt praktisch keinerlei störende Rückwirkung vom Ventilsystem auf den Probanden ein und der Atemluft wird nur ein praktisch vernachlässigbarer Strömungswiderstand entgegengesetzt. Hierdurch wird die Genauigkeit und Zuverlässigkeit der Messungen wesentlich verbessert.

**Patentansprüche**

1. Gerät zur Untersuchung der Lungenfunktion mit einem an die Atemwege einer zu untersuchenden Person anschließbaren Atemrohr (10), das mit einem Strömungssensor (56, 58) gekoppelt und über ein Ventil (22, 24, 26, 28), welches eine Offenstellung und eine Schließstellung aufweist, an eine Atemluftleitung (30, 32, 34 oder 36) angeschlossen ist, dadurch gekennzeichnet, daß die Offenstellung des Ventils durch einen Drucksensor (18), der auf den Druck im Atemrohr (10) anspricht, beim Überschreiten eines vorgegebenen Mindestdruckwertes eingestellt wird und die Schließstellung durch den Strömungssensor (56, 58) beim Unterschreiten eines vorgegebenen Strömungswertes eingestellt wird.

2. Gerät nach Anspruch 1, dadurch gekennzeichnet, daß der Drucksensor (18) und der Strömungssensor (56, 58) ein Setz- bzw. Rücksetzsignal an eine bistabile Schaltung (62 bzw. 64) liefern, die ein das Ventil betätigendes Signal (IN, EX) liefert.

3. Gerät nach Anspruch 1 oder 2, dadurch gekennzeichnet, daß das Ventil pneumatisch betätigbar ist.

4. Gerät nach Anspruch 1, 2 oder 3, bei dem das Atemrohr mit einem quer zu ihm verlaufenden Rohr verbunden ist, in dem sich das Ventil befindet, dadurch gekennzeichnet, daß die Atemluftleitung (36a, 36b) von dem quer verlaufenden Rohr (74, 76) im Abstand von der Mündung des Atemrohrs (10) quer abzweigt und daß sich zwischen der Mündung des Atemrohrs (10) und der Abzweigstelle der Atemluftleitung (36a oder 36b) ein Ventilsitz (78a bzw. 78b) des Ventils angeordnet ist, welcher mit einem Schließkörper (80a bzw. 80b) zusammenwirkt, der längs des quer verlaufenden Rohres (74) beweglich gelagert ist.

5. Gerät nach Anspruch 4, dadurch gekennzeichnet, daß das Atemrohr (10) mit zwei sich an seiner Mündung kreuzenden quer zu ihm verlaufenden Rohren (74, 76) verbunden ist, an das zwei weitere Atemluftleitungen (36c, 36d) über jeweils ein Ventil angeschlossen sind.

6. Gerät nach einem der vorhergehenden Ansprüche, dadurch gekennzeichnet, daß der Drucksensor (18) eine leicht bewegliche, biegsame Membrane (102) enthält, deren eine Seite an einen mit dem Atemrohr (10) verbundenen Raum angrenzt und deren andere Seite mit einem Steuerhebel (106) verbunden ist, daß der Steuerhebel durch eine Federanordnung (112, 114) in eine Ruhelage vorgespannt ist, daß das eine Ende des Hebels in einem reibungsarmen, leicht beweglichen Schwenklager (108) gelagert ist und daß am anderen Ende des Steuerhebels ein Permanentmagnet (110) angeordnet ist, der mit einer Hallsonden-Sensoranordnung (116, 118) zusammenarbeitet.

**Claims**

1. Pulmonary function evaluation device having a breath tube (10) which may be connected to the respiratory tracts of a person to be examined, which tube is coupled to a flow sensor (56, 58) and is connected by means of a valve (22, 24, 26, 28) which has an open position and a closed position to a ventilating air line, characterised in that the open position of the valve is set by a pressure sensor (18) which is responsive to the pressure in the breath tube (10) when a predetermined minimum pressure value is exceeded and the closed position is set by the flow sensor (56, 58) when the flow is less than a predetermined value.

2. Device as claimed in claim 1, characterised in that the pressure sensor (18) and the flow sensor (56, 58) deliver a setting and re-setting signal respectively to a bistable circuit (62 or 64) which delivers a signal (IN, EX) actuating the valve.

3. Device as claimed in claim 1 or 2, characterised in that the valve is pneumatically actuable.

4. Device as claimed in claim 1, 2 or 3 in which the breath tube is connected to a tube extending transverse to it in which the valve is situated, characterised in that the ventilating air line (36a, 36b) branches off transversely from the transversely extending tube (74, 76) at a distance from the mouth of the breath tube (10) and that between the mouth of the breath tube (10) and the branching point of the ventilating air line (36a or 36b) a valve seat (78a or 78b) of the valve is disposed which cooperates with a closure member (80a or 80b) which is mounted to move in the direction of the length of the transversely extending tube (74).

5. Device as claimed in claim 4, characterised in that the breath tube (10) is connected to two tubes (74, 76) crossing at its mouth and extending transverse to it, to which two further ventilating air lines (36c, 36d) are connected via a respective valve.

6. Device as claimed in one of the preceding claims, characterised in that the pressure sensor (18) includes a readily movable, flexible membrane (102), of which one side borders a space connected to the breath tube (10) and whose other side is connected to a control lever (106) that the control lever is biassed into a rest position by a spring arrangement (112, 114), that one end of the lever is mounted in a low-friction, readily movable pivotal bearing (108) and that at the other end of the control lever a permanent magnet (110) is disposed which cooperates with a Hall probe sensor arrangement (116, 118).

**Revendications**

1. Dispositif d'examen de la fonction pulmonaire comportant un tube de respiration (10) pouvant être raccordé aux voies respiratoires de la personne à examiner, lequel est couplé à un détecteur d'écoulement (56, 58) et est raccordé à une canalisation d'air de respiration (30, 32, 34 ou 36) par l'intermédiaire d'un clapet (22, 24, 26, 28), qui comporte une position d'ouverture et une position de fermeture, caractérisé par le fait que la position ouverte du clapet est réglée, lors du dépassement d'une valeur minimale de pression prédéterminée, à l'aide d'un détecteur de pression (18) qui réagit à la pression dans le tube de respiration (10), et la position fermée est réglée lorsqu'on passe en dessous d'une valeur d'écoulement prédéterminée, à l'aide du détecteur d'écoulement (56, 58).

2. Dispositif selon la revendication 1, caractérisé par le fait que le détecteur de pression (18) et le détecteur d'écoulement(56, 58) fournissent un signal de mise à un et de mise à zéro à un circuit bistable (62, 64) qui fournit un signal (IN, EX) actionnant le clapet.

3. Dispositif selon la revendication 1 ou 2, caractérisé par le fait que le clapet peut être actionné pneumatiquement.

4. Dispositif selon la revendication 1, 2 ou 3, dans lequel le tube de respiration est relié à un tube s'étendant transversalement à lui, dans lequel se trouve le clapet, caractérisé par le fait que la canalisation d'air de respiration (36a, 36b) bifurque transversalement à partir du tube s'étendant transversalement (74, 76), à une certaine distance de l'embouchure du tube de respiration (10), et qu'on place entre l'embouchure du tube de respiration (10) et le point de bifurcation de la canalisation d'air de respiration (36a, 36b) un siège (78a, 78b) du clapet, qui coopère avec un corps de fermeture (80a, 80b) qui est monté de façon à pouvoir se déplacer le long du tube (74) s'étendant transversalement.

5. Dispositif selon la revendication 4, caractérisé par le fait que le tube de respiration (10) est relié à deux tubes (74, 76) s'étendant transversalement à lui et se croisant à son embouchure, auxquels sont raccordés chacune par l'intermédiaire d'un clapet, deux autres canalisations d'air de respiration (36c, 36d).

6. Dispositif selon l'une des revendications précédentes, caractérisé en ce que le détecteur de pression (18) contient une membrane flexible (102) facilement mobile dont un côté est adjacent à un volume relié au tube de respiration (10) et dont l'autre côté est relié à un levier de commande (106), que le levier de commande est précontraint dans une position de repos par un agencement de ressorts (112, 114), que l'une des extrémités du levier est montée dans un palier de pivotement (108), facilement mobile et pratiquement sans frottement, et qu'à l'autre extrémité du levier de commande est disposé un aimant permanent (110) qui coopère avec un dispositif de détection à sonde de Hall (116, 118).

# Fig.1

# Fig. 2

## Fig. 3

## Fig. 4